# EUROPEAN PATENT APPLICATION

(11) **EP 4 458 435 A1**
(43) Date of publication of application: **06.11.2024**
(21) Application number: 22916653.3
(22) Date of filing: 26.12.2022
(51) Int. Cl.: A63B 24/00, A63B 71/06, A61B 5/11, A61B 5/22

(54) **AI EXERCISE GUIDE DEVICE AND METHOD**

(30) Priority: 28.12.2021 KR 20210190367; 28.12.2021 KR 20210190368; 23.12.2022 KR 20220183460
(71) Applicant: DRAX Inc., Anyang-si, Gyeonggi-do 14086 (KR)
(72) Inventor: YOO, Seon Kyung, Seoul 08251 (KR)
(74) Representative: Mewburn Ellis LLP
(86) International application number: PCT/KR2022/021299
(87) International publication number: WO 2023/128512

(57) **Abstract**

An artificial intelligence (Al) workout guide device includes a storage storing one or more muscles activated when fitness equipment is used, a contribution of each of the one or more muscles, and an workout trajectory guide generated when accurately using the fitness equipment in a correct posture, a sensor configured to detect an workout trajectory corresponding to movement of a user moving the fitness equipment, an AI processor configured to determine whether a deviation between the workout trajectory detected by the sensor when the fitness equipment is moved in at least one direction matches the workout trajectory guide, and a workout guider configured to, when the deviation between the workout trajectory and the workout trajectory guide exceeds a preset value, guide to adjust an workout load of the fitness equipment or an workout posture.

## Description

### Technical Field

The present disclosure relates to the provision of a user with information about muscle contraction and relaxation when the user works out.

### Background Art

Weight fitness equipment is provided in various forms according to a purpose of use or a body part, in order to increase muscle strength, and is configured to train the upper body and the lower body by mainly using the hands or feet. A user may work out by moving a selected weight through a fitness structure of fitness equipment.

However, it is difficult for a user to determine whether they are appropriately using the fitness equipment according to their physical characteristics, workout capability, or the purpose of the fitness equipment.

### Disclosure

### Technical Problem

An embodiment of the present disclosure provides in real time a workout guide including information about muscle contraction and relaxation when a user works out using fitness equipment, thereby improving a workout effect.

An embodiment of the present disclosure provides in real time a workout guide including breathing information when a user works out using fitness equipment, thereby improving a workout effect.

### Technical Solution

According to an embodiment of the present disclosure, there is provided an artificial intelligence (Al) workout guide device including a storage storing one or more muscles activated when fitness equipment is used, a contribution of each of the one or more muscles, and a workout trajectory guide generated when the fitness equipment is used accurately with a correct posture, a sensor configured to detect a workout trajectory corresponding to movement of a user of moving the fitness equipment, an AI processor configured to determine whether a deviation between the workout trajectory detected by the sensor when the fitness equipment is moved in at least one direction matches the workout trajectory guide, and a workout guider configured to guide to adjust a workout load of the fitness equipment or a workout posture when the deviation between the workout trajectory and the workout trajectory guide exceeds a preset value.

### Advantageous Effects

According to an embodiment of the present disclosure, the artificial intelligence (Al) workout guide method may provide in real time the workout guide including the information about muscle contraction and relaxation when the user works out using the fitness equipment, thereby improving a workout effect.

According to an embodiment of the present disclosure, the AI workout guide method may provide in real time the workout guide including the breathing information when the user works out using the fitness equipment, thereby improving a workout effect.

According to an embodiment of the present disclosure, the AI workout guide method may provide the workout guide to the user when the user's workout posture is incorrect or the weight of the fitness equipment used by the user is unsuitable for the user based on the workout trajectory information obtained when the user uses the fitness equipment, thereby improving a workout effect.

### Description of Drawings

FIG. 1 illustrates a smart gym system using an artificial intelligence (Al) workout guide device, according to an embodiment of the present disclosure.
FIG. 2 illustrates an example of fitness equipment provided in a smart gym and implementing an AI workout guide method, according to an embodiment of the present disclosure.
FIG. 3 is a block diagram of an internal configuration of a smart gym system, according to an embodiment of the present disclosure.
FIG. 4 illustrates an example of classifying workout trajectory big data, according to an embodiment of the present disclosure.
FIG. 5 illustrates an example in which a display displays one or more muscles activated when working out and a breathing guide.
FIG. 6 illustrates an example of a workout trajectory detected by fitness equipment, according to an embodiment of the present disclosure.
FIG. 7 illustrates an example in which a display displays in real time one or more muscles activated when working out and a breathing guide.

### Best Mode

According to an embodiment of the present disclosure, there is provided an artificial intelligence (Al) workout guide device including a storage storing one or more muscles activated when fitness equipment is used, a contribution of each of the one or more muscles, and a workout trajectory guide generated when the fitness equipment is used accurately with a correct posture, a sensor configured to detect a workout trajectory corresponding to movement of a user of moving the fitness equipment, an AI processor configured to determine whether a deviation between the workout trajectory detected by the sensor when the fitness equipment is moved in at least one direction matches the workout trajectory guide, and a workout guider configured to guide to adjust a workout load of the fitness equipment or a workout posture when the deviation between the workout trajectory and the workout trajectory guide exceeds a preset value.

The AI workout guide device may further include a display configured to, based on the contribution, display a load applied to each of the one or more muscles activated when a user uses the fitness equipment.

The AI workout guide device may further include a display configured to display in real time information about a state of contraction or relaxation of each of the one or more muscles activated when the fitness equipment is used.

The display may display in real time a contraction guide or relaxation guide with respect to a major muscle activated when a user uses the fitness equipment, based on the workout trajectory detected by the sensor and the workout trajectory guide.

The AI workout guide device may further include a display configured to display, as indicators, an exhalation in a direction in which a major muscle contracts and an inhalation in a direction in which the major muscle relaxes, among the one or more muscles activated when the fitness equipment is used.

The sensor may detect an angle of a joint when the user moves the fitness equipment.

The AI workout guide device may further include a display configured to, based on the detected angle of the joint, display about a state of contraction or relaxation of each of the one or more muscles activated when the fitness equipment is used.

The workout guider may guide a workout posture to be adjusted when a shape of a workout trajectory corresponding to the angle of the joint in a certain range detected by the sensor is different from a shape of the workout trajectory of the previously stored workout trajectory guide.

According to an embodiment of the present disclosure, there is provided an AI workout guide method including detecting, by a sensor, a workout trajectory corresponding to movement of a user of moving fitness equipment, determining, an AI processor, whether the workout trajectory detected by the sensor when the fitness equipment is moved in at least one direction matches a workout trajectory guide previously stored in a storage and generated when the fitness equipment is used appropriately according to usage standards, and guiding, by a workout guider, a workout load of the fitness equipment or a workout posture to be adjusted when the deviation between the workout trajectory detected and the workout trajectory guide exceeds a preset value.

The AI workout guide method may further include based on the contribution, displaying, by the display, a load applied to each of the one or more muscles activated when a user uses the fitness equipment.

The AI workout guide method may further include displaying in real time, by the display, information about a state of contraction or relaxation of each of the one or more muscles when the fitness equipment is used.

The AI workout guide method may further include displaying as indicators, by the display, an exhalation in a direction in which a major muscle contracts and an inhalation in a direction in which the major muscle relaxes, among the one or more muscles activated when the fitness equipment is used.

The AI workout guide method may further include further detecting, by the sensor, an angle of a joint when the user moves the fitness equipment, and, based on the detected angle of the joint, displaying in real time, by the display, information about a state of contraction or relaxation of each of the one or more muscles activated when the fitness equipment is used.

### Mode for Invention

Hereinafter, some embodiments of the present disclosure will be described in detail with reference to the attached drawings in order to enable one of ordinary skill in the art to easily practice the present disclosure.

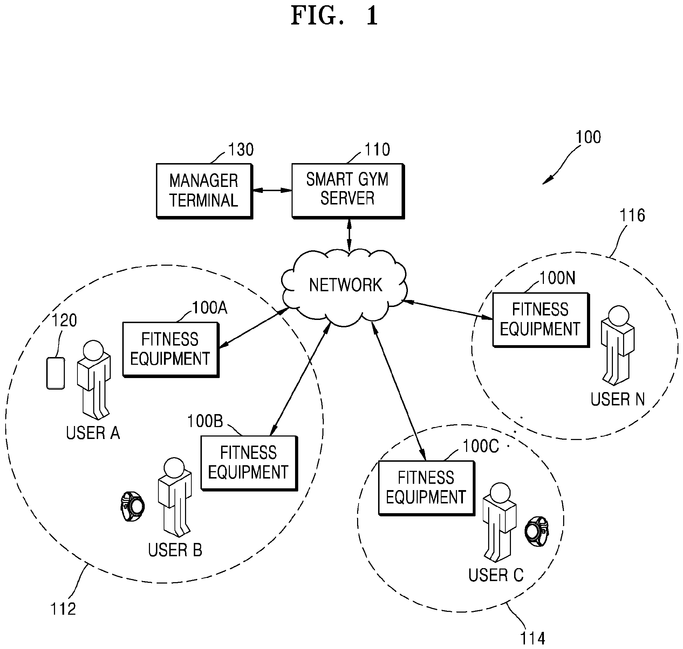

FIG. 1 illustrates a smart gym system using an artificial intelligence (Al) workout guide device, according to an embodiment of the present disclosure.

A smart gym system 100 includes a smart gym server 110, at least one of pieces of fitness equipment 100a through 100n, at least one user terminal 120, and a manager terminal 130.

In FIG. 1, the smart gym server 110 may communicate with a first smart gym 112, a second smart gym 114, and an nth smart gym 116, which are physically at different locations, and may transmit or receive data to or from at least one of the pieces of fitness equipment 100a and 100b provided in the first smart gym 112, at least one piece of the fitness equipment 100c provided in the second smart gym 114, and at least one piece of the fitness equipment 100n provided in the nth smart gym 116.

According to an embodiment of the present disclosure, a smart gym denotes a physical space that enables a user to provide an athletic performance using fitness equipment to the smart gym server 110, and the smart gym server 110 to learn and analyze the athletic performance of the user and provide workout prescription suitable to the user. The smart gym may be realized as a fitness center, a gym, or a space including fitness equipment.

Users USER A through USER N who come to the smart gym to workout may enter the smart gym after identification when entering the smart gym. For example, the user may enter the smart gym through member verification by tagging the user terminal 120 to an unmanned terminal, such as a kiosk, at an entrance of the smart gym via near field communication (NFC) or radio frequency identification (RFID), or enter the smart gym after member verification at the unmanned terminal through bio-information identification, such as face recognition.

Information about the user who has been verified as a member may be transmitted from the smart gym server 110 to at least one of the pieces of fitness equipment 100a through 100n through a network. For example, the smart gym server 110 may transmit the information about the user to fitness equipment to which the user tagged the user terminal 120. According to an embodiment of the present disclosure, the information about the user is also referred to as user data, and may include at least some or all of a gender, age, weight, height, body mass index (BMI), and body fat percentage.

The smart gym server 110 may guide the users USER A and USER B who use the fitness equipment 100a and 100b provided in the first smart gym 112, respectively, with a workout guide such as a workout method, a workout intensity, a breathing method, a workout posture, etc. to each user. Also, the smart gym server 110 may provide values, such as a target weight and a recommended usage velocity, of the fitness equipment 100a and 100b. Also, the smart gym server 110 may receive athletic performances of the users USER A and USER B using the fitness equipment 100a and 100b, respectively. The smart gym server 110 may further receive, from the user terminal 120, log information or health information, such as a user's heart rate, blood pressure, and pulse.

The smart gym server 110 may be realized in a form of a cloud server. The smart gym server 110 may combine pieces of information collected from pieces of fitness equipment in smart gym fitness centers at different locations, and manage the pieces of information. For example, the smart gym server 110 may combine a history of a first user using fitness equipment in the first smart gym 112 at a first location and a history of the first user using fitness equipment in the second smart gym 114 at a second location, and manage the histories.

According to an embodiment of the present disclosure, the at least one of the pieces of fitness equipment 100a through 100n may be stretching fitness equipment, weight fitness equipment, or aerobic fitness equipment. The at least one of the pieces of fitness equipment 100a through 100n may provide an appropriate workout guide to a user through a display attached thereto or a display capable of communicating therewith via wires or wirelessly. For example, the stretching fitness equipment provides a workout guide related to stretching to be performed by a user, through a smart mirror capable of communicating with the stretching fitness equipment. However, an embodiment is not limited thereto, and a workout guide may be provided by using any one of various output methods, such as a speaker or vibration.

At least one of the pieces of fitness equipment 100a through 100n may perform wired/wireless communication with the smart gym server 110, the user terminal 120, and the manager terminal 130.

According to an embodiment of the present disclosure, the user terminal 120 may be realized in a form of a smart phone, a smart watch, a handheld device, or a wearable device. Also, an application for using the smart gym system may be installed in the user terminal 120. The user terminal 120 may receive workout sequence information from the smart gym server 110. A workout sequence denotes a workout plan planned considering strength and workout capability of a user. The workout sequence includes information about a fitness equipment list to be used by the user, a target weight of each piece of fitness equipment, and the number of times each piece of fitness equipment is used.

When using at least one of the pieces of fitness equipment 100a through 100n in the smart gym system 100, a user may perform communication by tagging the user terminal 120 through NFC or RFID, or perform identification by using a physical characteristic of the user. When the identification of the user is completed, the smart gym server 110 may transmit user data to fitness equipment tagged by the user.

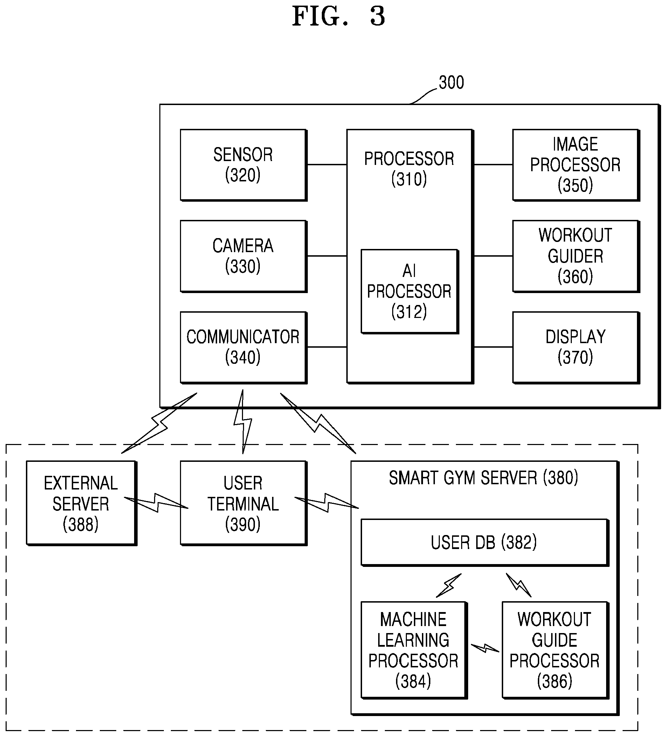

FIG. 3 is a block diagram of an internal configuration of a smart gym system implementing an AI workout guide method, according to an embodiment of the present disclosure. The smart gym system may include an AI workout guide device 300 and a smart gym server 380.

The AI workout guide device 300 may communicate with the smart gym server 380, a user terminal 390, and an external server 388. The AI workout guide method may be implemented in fitness equipment or the smart gym server 380 within the smart gym system.

According to an embodiment of the present disclosure, the AI workout guide device 300 may include a processor 310, a sensor 320, a communicator 340, a workout guider 360, and a display 370. Also, the AI workout guide device 300 may further include storage (not shown), a camera 330 and an image processor 350. The processor 310 may include an AI processor 312 when necessary. The AI processor 312 may detect a workout trajectory based on data sensed by the sensor 320 and analyze a difference between the detected workout trajectory and a workout trajectory guide. In an embodiment of the present disclosure, functions of the AI processor 312 may also be implemented in a workout guide processor 386 of the smart gym server 380. The storage may receive and store necessary data through communication with the smart gym server 380, the user terminal 390, or the external server 388. In addition, the AI workout guide device 300 may store in the storage or receive through communication with the smart gym server 380 one or more muscles activated when the fitness equipment is used, a contribution of each of the one or more muscles, and a workout trajectory guide generated when the fitness equipment is used accurately with a correct posture.

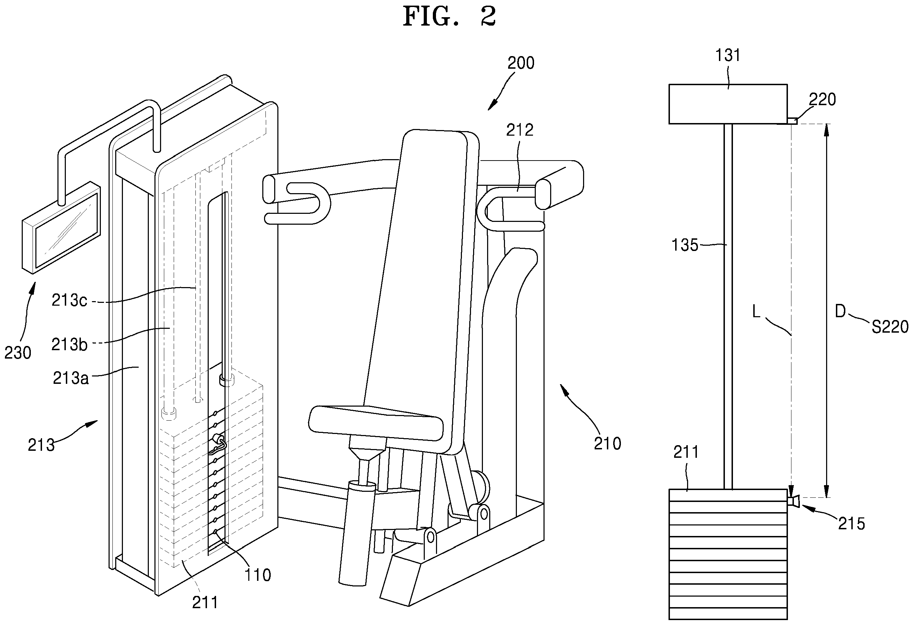

FIG. 2 illustrates a shoulder press machine 200 that is an example of fitness equipment in which the AI workout guide device 300 is installed.

A sensor 220 may be installed at a frame structure 213 of a fitness body 210. The frame structure 213 includes a base frame 213a, a guide rail 213b, and a connection line 213c. The sensor 220 irradiates a laser beam towards a pin structure 215, receives a reflected laser beam, and measures a distance D S220 from the sensor 220 to the pin structure 215 in real time or in units of pre-set t times. Accordingly, the sensor 220 may detect, in real time, at least one of a location, a moving velocity, and a moving direction of a weight member 211 selected by the pin structure 215. Also, when a user moves a weight plate by pushing a handle 212 of the shoulder press machine 200, the sensor 220 may measure the distance D S220 to the pin structure 215 embedded in the weight plate and detect a workout trajectory based on the distance D S220. The sensor 220 may also be attached to the shoulder press machine 200 and detect an angle of the joint used when the user uses the shoulder press machine 200. In this case, a mark, etc. indicating a reference point may be attached to the user's joint. The sensor 220 may detect the angle of the joint in real time by sensing the mark attached to the joint. However, this is only an example and it should be noted that various modifications are possible. A display 230 may display a state of contraction or relaxation of each of one or more muscles activated when the user uses the shoulder press machine 200, and also display a load applied to each of the one or more muscles. The display 230 may also display in real time a contraction guide or relaxation guide with respect to a major muscle activated when the user uses the shoulder press machine 200. Also, the display 230 may further display a breathing guide.

In an embodiment of the present disclosure, the state of contraction or relaxation of each of the one or more muscles activated when the user uses the shoulder press machine 200 refers to a state of contraction or relaxation of the user's muscle detected by the sensor 220. In an embodiment of the present disclosure, the contraction guide or relaxation guide refers to a muscle in which contraction occurs and a contraction time, and a muscle in which relaxation occurs and a relaxation time when the user uses the shoulder press machine 200 in a correct posture and in accordance with usage standards.

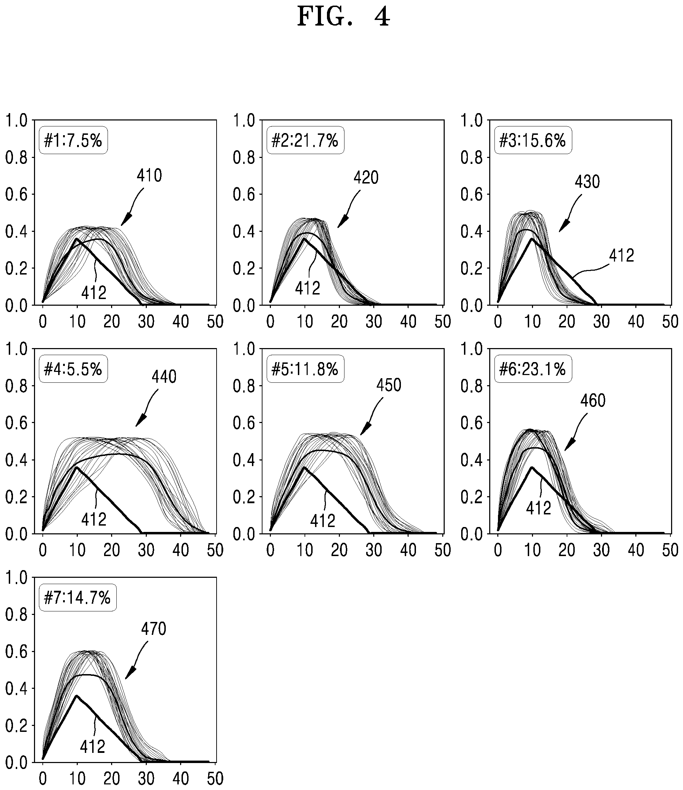

The AI processor 312 determines whether the workout trajectory detected by the sensor 320 matches the workout trajectory guide received from the smart gym server. In addition, the AI processor 312 determines whether the workout trajectory corresponding to the angle of the joint matches the workout trajectory guide. For the workout trajectory guide, refer to the description in FIG. 4.

For example, when the user uses a seated leg curl machine, the AI processor 312 determines whether a workout trajectory in a range where a knee joint is 0 to 4 ° matches the workout trajectory guide. Referring to Table 5, the range where the knee joint is 0 to 4 ° is a range where the gastrocnemius is activated. In addition, when the user uses the seated leg curl machine, the AI processor 312 determines whether a workout trajectory in a range where the knee joint is 5 ° to 90 ° matches the workout trajectory guide. Referring to Table 5, the range where the knee joint is 5 ° to 90 ° is a range where the biceps femoris, semitendinosus, and semimembranosus are activated.

The AI processor 312 determines that it is necessary to adjust the workout posture when a shape of the workout trajectory corresponding to the angle of the joint in a certain range detected by the sensor 220 is different from a shape of the workout trajectory guide. In this case, the AI processor 312 may further refer to a workout posture of the user detected by the image processor 350.

For example, when a discrepancy occurs in the range where the knee joint is 0 to 4 °, the AI processor 312 determines that there is a problem with the user's gastrocnemius or that there is a problem with a workout posture related to the gastrocnemius. When a discrepancy occurs in the range where the knee joint is 5 ° to 90 °, the AI processor 312 determines that there is a problem with the biceps femoris, semitendinosus, and semimembranosus, or that there is a problem with a workout posture related to the biceps femoris, semitendinosus, and semimembranosus.

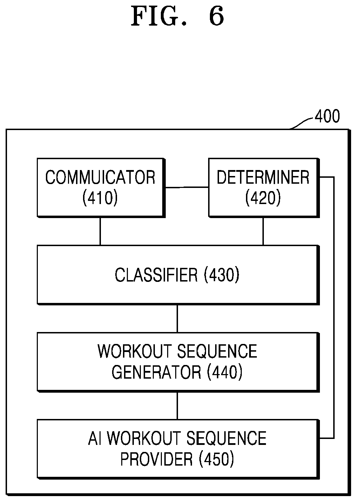

The AI processor 312 also determines that it is necessary to adjust the magnitude of the workout load when a deviation between a movement displacement of the workout trajectory corresponding to the angle of the joint in the certain range detected by the sensor 220 and a movement displacement of a height or a width of the workout trajectory guide received from the smart gym server 380 exceeds a preset value. For example, referring to FIG. 6, when a height H 612 of the user's workout trajectory detected by the sensor 320 is lower than a height h of a previously stored workout trajectory guide by a preset ratio or more, the AI processor 312 determines that it is necessary to reduce the magnitude of the workout load.

The communicator 340 may receive a user input through the display 230 or may transmit or receive user data to or from a user DB 382 of the smart gym server 380. The communicator 340 may also communicate with the external server 388.

The workout guider 360 may provide the user with information such as user data, the target weight of the fitness equipment, the workout trajectory guide, a movement speed guide of the fitness equipment, the breathing guide, and the contraction guide or relaxation guide with respect to the major muscle activated when the fitness equipment is used received from the smart gym server 380.

The workout guider 360 may provide a workout guide based on determination results of the AI processor 312. The workout guider 360 may guide the AI processor 312 to reduce or increase the workout load of the fitness equipment when the deviation between the workout trajectory detected by the sensor 320 and the workout trajectory guide exceeds the preset value. In addition, when the AI processor 312 determines that the shape of the workout trajectory detected by the sensor 320 is different from the shape of the workout trajectory guide, the workout guider 360 may guide the AI processor 312 to that it is necessary to change the workout posture.

The smart gym server 380 includes a user DB 382, a machine learning processor 384, and the workout guide processor 386. The user DB 382 stores and manages the user data. The user data includes a gender, age, weight, height, BMI, and body fat percentage. The machine learning processor 384 analyzes, accumulates, learns, and processes big data including the user data acquired from the user DB, workout records of fitness equipment used by users using the smart gym, etc.

According to an embodiment of the present disclosure, the smart gym server 380 may provide the AI workout guide method by receiving data obtained from the sensor 220 installed in the fitness equipment. The smart gym server 380 stores in a storage (not shown) the one or more muscles activated when fitness equipment is used, the contribution of each of the one or more muscles, and the workout trajectory generated when the fitness equipment is used appropriately for usage standards. Also, the workout guide processor 386 may determine whether the workout trajectory obtained from received sensing data matches the workout trajectory guide and then guide the user to adjust the workout load of the fitness equipment used by the user or the workout posture.

According to an embodiment of the present disclosure, the smart gym server 380 includes a mapping table storing personal maximum weight (PMW)ₑₛₜᵢₘₐₜᵢₒₙ percentile information obtained based on PMW data obtained from a population per fitness equipment as shown in Table 3 below. Table 1 shows the PMWₑₛₜᵢₘₐₜᵢₒₙ percentile information with respect to a leg extension machine.

**[Table 1]**

| PMWₑₛₜᵢₘₐₜᵢₒₙ percentile value | Population PMWₑₛₜᵢₘₐₜᵢₒₙ (kg) |
|---|---|
| PMW90 | 103.3 |
| ... | ... |
| PMW50 | 67.3 |
| PMW40 | 66.5 |
| ... | ... |
| PMW10 | 24.6 |

According to an embodiment of the present disclosure, a PMW denotes muscular strength that may be exerted by an individual against a resistance of a weight with an utmost effort. Examples of the PMW include 1 RM and 4 RM. The PMWₑₛₜᵢₘₐₜᵢₒₙ indicates a value obtained by estimating a PMW of the user, based on an estimated muscular strength value calculated through Equation 1. The smart gym server 380 may determine PMWₑₛₜᵢₘₐₜᵢₒₙ per fitness equipment. The smart gym server 380 may determine PMWₑₛₜᵢₘₐₜᵢₒₙ of the fitness equipment to be used by the user based on the estimated muscular strength value and the PMWₑₛₜᵢₘₐₜᵢₒₙ percentile information. Also, the smart gym server 380 may automatically set an initial target weight of the fitness equipment to be used by the user based on the PMWₑₛₜᵢₘₐₜᵢₒₙ.

For example, when an estimated muscular strength value of a 32-year-old man is 55.9 and a percentile value to which the estimated muscular strength value belongs is 40, the smart gym server 380 estimates a corresponding PMWₑₛₜᵢₘₐₜᵢₒₙ percentile value of 40, i.e., 66.5 kg, as a leg extension PMW of the 32-year-old man. According to an embodiment of the present disclosure, the smart gym server 380 may previously store the PMWₑₛₜᵢₘₐₜᵢₒₙ percentile information per fitness equipment as shown in Table 1.

The workout guide processor 386 may set an initial target weight per fitness equipment based on the PMWₑₛₜᵢₘₐₜᵢₒₙ. In this case, the workout guide processor 386 may set the initial target weight according to a workout intensity or a workout purpose.

The workout guide processor 386 may set the weight as a % of the PMWₑₛₜᵢₘₐₜᵢₒₙ when the workout intensity is a low intensity, b % of the PMWₑₛₜᵢₘₐₜᵢₒₙ when the workout intensity is a medium intensity, and c % of the PMWₑₛₜᵢₘₐₜᵢₒₙ when the workout intensity is a high intensity. For example, a may be set to 20 to 40, b may be set to 40 to 60, and c may be set to 60 to 80. However, this is only an example and it should be noted that various modifications are possible.

The workout guide processor 386 may provide the initial target weight of the fitness equipment to be used by the user to the workout guider 360 of the AI workout guide device 300, and the workout guider 360 may display the initial target weight of the fitness equipment 300 to be used by the user on the display 370. The display 370 includes a display.

According to another embodiment of the present disclosure, the smart gym server 380 may predict a PMW_{individual} that is a maximum muscular strength value reflecting an individual objectification index of the user. In addition, the smart gym server 380 may update the target weight of the fitness equipment based on the predicted PMW_{individual}. More specifically, the smart gym server 380 sets the initial target weight of the fitness equipment based on the PMWₑₛₜᵢₘₐₜᵢₒₙ, when the individual objectification index of the user is obtained for a preset period of time, reflects the individual objectification index of the user, and predict the PMW_{individual}. Thereafter, the smart gym server 380 may update the target weight of the fitness equipment based on the PMW_{individual}. The workout guide processor 386 may display the target weight updated based on the PMW_{individual} on the fitness equipment to be used by the user.

Examples of the individual objectification index include an athletic performance, a weight of fitness equipment determined when the fitness equipment is used, the number of repetitions (reps), the number of sets, a workout trajectory, a moving velocity, and regularity of reps in units of sets.

The machine learning processor 384 learns and processes the individual objectification index including athletic performances of the user on pieces of fitness equipment used in the smart gym. The machine learning processor 384 may update the PMWₑₛₜᵢₘₐₜᵢₒₙ to a PMW_{individual}, based on the individual objectification indexes of the pieces of fitness equipment used by the user in the smart gym for a certain period of time. The machine learning processor 384 may update the PMW_{individual} to a value greater than PMWₑₛₜᵢₘₐₜᵢₒₙ when the individual objectification index is a first reference value or more, and update the PMW_{individual} to a value less than the PMWₑₛₜᵢₘₐₜᵢₒₙ when the individual objectification index is a second reference value or less.

Accordingly, the machine learning processor 384 may learn and predict PMW_{individual} appropriate to a first user and a second user, who have a same gender, age, weight, height, BMI, and body fat percentage, for each piece of fitness equipment used by the first user and the second user by further reflecting individual objectification indexes including athletic performances of the first user and the second user, even when PMWₑₛₜᵢₘₐₜᵢₒₙ of the first user and the second user are estimated to be the same.

According to an embodiment of the present disclosure, the machine learning processor 384 may learns and processes the workout trajectory detected by fitness equipment. This will be described with reference to FIG. 6. The machine learning processor 384 may determine completion of the workout trajectory by using at least some of an ascent starting point 611, a descent starting point 613, an ascending section velocity V1 S610, a descending section velocity V2 S620, an ascending section average velocity, a descending section average velocity, and the height H 612, which are determined from the workout trajectory detected while the user uses fitness equipment, convert a degree of the completion of the workout trajectory into a numerical value, and use the numerical value as the individual objectification index. For example, the machine learning processor 384 determines that the ascent starting point 611 is appropriate when the ascent starting point 611 is 0 to tₐ seconds, and is late when the ascent starting point 611 exceeds tₐ seconds. Also, the completion may be rated good, fair, or bad or scored from 1 to 10, according to a pre-set standard for the appropriate ascent starting point 611. Similarly, according to a pre-set standard, when an ascending section velocity V1 S610 is between Va and Vb, it is determined that the ascending section velocity V1 S610 is appropriate, when the ascending section velocity V1 S610 exceeds Vb, it is determined that the ascending section velocity V1 S610 is fast, and when the ascending section velocity V1 S610 is less than Va, it is determined that the ascending section velocity V1 S610 is slow. A result of determining appropriate, fast, or slow may be rated good, fair, or bad or scored from 1 to 10. In the same way as above, the machine learning processor 384 may generate the objectification index from the workout record of the user.

According to another embodiment of the present disclosure, the machine learning processor 384 may, when reps is used as the individual objectification index, determine completion of the reps based on regularity between workout trajectories of all reps configuring one set and a performance time during which all reps configuring one set has been performed, convert a degree of the completion of the reps into a numerical value, and use the numerical value as the individual objectification index.

According to another embodiment of the present disclosure, the machine learning processor 384 may provide a workout trajectory guide optimized for each user by classifying workout trajectory big data. This will be described with reference to FIG. 4.

FIG. 4 illustrates an example in which the machine learning processor 384 classifies workout trajectories obtained from the population with respect to specific fitness equipment into seven patterns 410 to 470. An x-axis denotes time and a y-axis denotes standardized movement displacement. The population is initially targeted at n preset people, but data of users using the smart gym may be continuously accumulated and used.

The machine learning processor 384 may classify the movement trajectory patterns of the population and determine a proportion of the population belonging to each of the seven patterns 410 to 470. The machine learning processor 384 selects a workout trajectory guide from among the seven analyzed workout trajectory patterns 410 to 470. In this case, the machine learning processor 384 selects the second workout trajectory pattern 420 and the sixth workout trajectory pattern 460, which have the smallest deviation from a preset reference value trajectory 412 with the highest workout effect, as the workout trajectory guide. The machine learning processor 384 may select one or more of the workout trajectory guide from the workout trajectory patterns 410 to 470 in consideration of differences in workout trajectories resulting from differences in height and physical conditions of each user. For example, referring to Fig.4, the machine learning processor 384 may select second workout trajectory pattern 420 and the sixth workout trajectory pattern 460 as the workout trajectory guide. The second workout trajectory pattern 420 corresponds to 21.7 % of the population, and the sixth workout trajectory pattern 460 corresponds to 23.1 % of the population.

The workout guide processor 386 selects the workout trajectory guide in which the detected workout trajectory of the user belongs to the closest pattern among the second workout trajectory pattern 420 and the sixth workout trajectory pattern 460 obtained from the machine learning processor 384. For example, when the movement displacement of the workout trajectory of the user is less than 0.5, the workout guide processor 386 selects the second workout trajectory pattern 420 and provides the second workout trajectory pattern 420 to the user as the workout trajectory guide. Alternatively, when the movement displacement of the workout trajectory of the user is about 0.6, the workout guide processor 386 selects the sixth workout trajectory pattern 460 and provides the sixth workout trajectory pattern 460 to the user as the workout trajectory guide.

The workout guide processor 386 may also determine a change in the workout trajectory of the user according to a workout load, the number of workout, and time. For example, when a first user uses a chest press machine of 60 kg, the workout trajectory belongs to the second workout trajectory pattern 420, but when the first user uses a chest press machine of 70 kg, the workout trajectory belongs to the first workout trajectory pattern 410, the workout guide processor 386 may determine that the load suitable for the first user is 60 kg. In this case, the workout guide processor 386 may guide the workout guider 360 to adjust the magnitude of the workout load. The configuration of the workout guide processor 386 may also be implemented in the AI processor 312.

According to another embodiment of the present disclosure, the smart gym server 380 stores information about fitness equipment corresponding to each body part, and joints and muscles used for each piece of fitness equipment in the storage, as shown in an example of Table 2. Table 2 shows an example of joint and muscle data for each piece of fitness equipment corresponding to the body part 'lower body'.

**[Table 2]**

| **Fitness equipment** | **Joint movement** | **Workout target muscle** | **Muscles** | |
|---|---|---|---|---|
| | | | **Major muscles** | **Minor muscles** |
| leg extension | knee extension | quadriceps femoris | | rectus femoris |
| | | | vastus lateralis oblique vastus medialis oblique | |
| | | | vastus intermedius | |
| seated leg curl | knee flexion | hamstring | biceps femoris | gastrocnemius |
| | | | semitendinosus | |
| | | | membranosus muscle | |
| leg press | knee extension | quadriceps femoris | rectus femoris | adductor transfer |
| | hip joint extension | gluteus medius | vastus lateralis oblique | hamstring |
| | | | vastus medialis oblique | gastrocnemius |
| | | | vastus intermedius | gastrocnemius |
| inner thigh | hip adduction | adductor longus | adductor longus | |
| | | adductor brevis | adductor brevis | |
| | | adductor magnus | adductor magnus | |
| | | gracilis | gracilis | |
| | | pectineus | pectineus | |
| out thigh | hip abduction | gluteus maximus | gluteus maximus | gluteus medius |

The smart gym server 380 also stores information about one or more muscles activated when fitness equipment is used and the contribution of each of the one or more muscles, as shown in Tables 3 and 4.

**[Table 3]**

| **Fitness equipment** | **Joint movement** | **Workout target muscles** | **Muscles** | |
|---|---|---|---|---|
| | knee extension | quadriceps femoris | major muscle (segmentation) | contribution |
| leg extension | | | rectus femoris | 20 % |
| | | | vastus lateralis oblique | 80 % |
| | | | vastus medialis oblique | |
| | | | vastus intermedius | |

**[Table 4]**

| **Fitness equipment** | **Joint movement** | **Workout target muscles** | **Muscles** | |
|---|---|---|---|---|
| seated leg curl | knee flexion | hamstring | major muscles (segmentation) | contribut ion |
| | | | biceps femoris | 60 % |
| | | | semitendinosus | 15 % |
| | | | semimembranosus | 15 % |
| | | | minor muscle | contribution |
| | | | gastrocnemius | 10 % |

The smart gym server 380 also stores information about one or more muscles used for each piece of fitness equipment and for each range of motion of joint, as shown in Table 5. In addition, the smart gym server 380 stores workout trajectories generated when the fitness equipment is used in accordance with the usage standards for each range of motion of joint, and provide the workout trajectories stored for each range of motion to the user as the workout trajectory guide. The workout trajectory guide refers to a workout trajectory generated when the fitness equipment is accurately used with a correct posture.

**[Table 5]**

| **Fitness equipment** | **Joint movement** | **Range of motion** | **Active muscle** | |
|---|---|---|---|---|
| | | | **Major muscle** | **Minor muscle** |
| leg extension | knee extension | 90 to 81 degrees | | rectus femoris |
| | | 80 to 5 degrees | vastus lateralis oblique vastus medialis oblique vastus intermedius | |
| | | 10 to 5 degrees | vastus medialis oblique | |
| seated leg curl | knee flexion | 0 to 4 degrees | | gastrocnemi us |
| | | 5 to 90 degrees | biceps femoris semitendinos us membranosu s muscle | |
| | | 50 to 90 degrees | biceps femoris | |

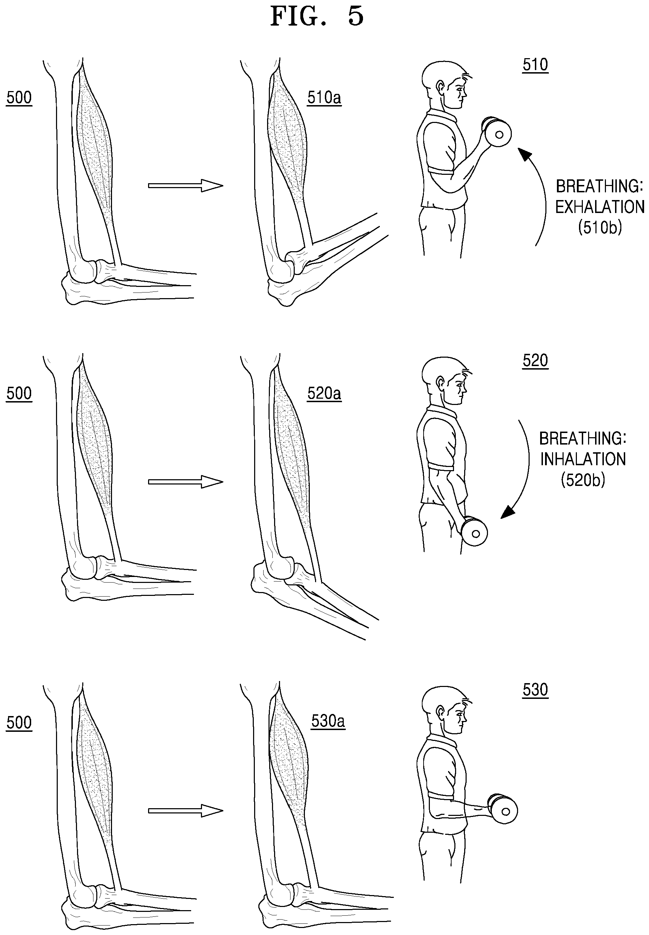

FIG. 5 illustrates an example in which a display displays one or more muscles activated when working out and a breathing guide.

FIG. 5 illustrates an example in which the user works on biceps by using a dumbbell. The display displays in real time information about a state of contraction 510a or relaxation 520a of a biceps brachii 500 which is activated when the dumbbell is used, based on an angle of a joint detected by a sensor. When the sensor detects the angle of an elbow joint and then a movement of folding the arm, the display displays the contraction 510a of the biceps brachii 500 based on the angle of the elbow joint, and displays a necessary breathing guide as an indicator 510b. In addition, when the sensor detects a movement of returning the arm to its original position after detecting the angle of the elbow joint and the movement of folding the arm, the display displays the relaxation 520a of the biceps brachii 500 based on the angle of the elbow joint, and displays a necessary breathing guide as an indicator 520b.

The breathing guide may be provided in various ways depending on fitness equipment or a workout method. For example, when the load is placed on the user's muscle, the exhalation 510b may be provided as the breathing guide, and when the load on the muscle is reduced, the inhalation 520b may be provided as the breathing guide. Whether the load is placed on the user's muscle may be determined from a workout trajectory.

According to another embodiment of the present disclosure, when a workout curve detected when the user works on biceps by using the dumbbell does not match a workout trajectory guide, an AI processor determines that the user's elbow joint is injured or has a problem. In this case, a workout guider may guide the user a workout method 530 of supporting the weight of the dumbbell without using the elbow joint.

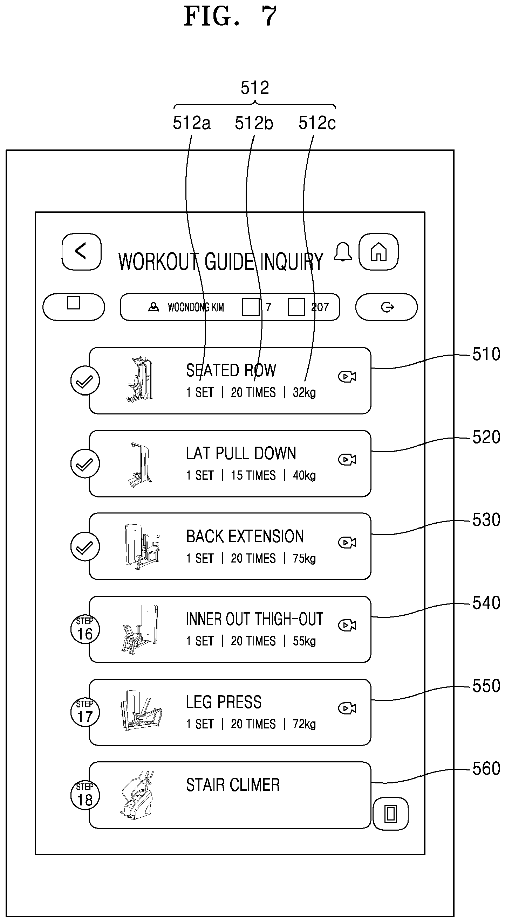

FIG. 7 illustrates an example in which a display displays in real time one or more muscles activated when working out and a breathing guide.

Assuming an example in which the user works on a leg extension machine 700 according to an initial target weight of 20 kg, the number of repetitions of 20 times, Reps of 1 time, and a workout sequence. An AI processor may calculate the amount of workout in real time based on a contribution of each of the one or more muscles activated when the user works out. A display 710 may display the amount of workout or accumulated amount of workout added to one or more muscles.

For example, in the case of 20 kg and the number of repetitions of 2, the AI processor calculates the accumulated amount of workout of rectus femoris as 20kg*2*20 %=8 kg 740b in a range where an angle of the user's knee joint is 90 ° to 81 °. In this case, a 'rectus femoris 730a', which is an activated muscle, is displayed in color or shade on the display 710. The display 710 may display the activated muscle in two or three dimensions. The user may intuitively understand the currently activated muscle by looking at the display 710. Also, in a range where the angle of the user's knee joint is 80 ° to 5 °, the accumulated amount of workout of vastus lateralis oblique, vastus medialis oblique, and vastus intermedius is calculated as 20kg*2*80 %. In addition, the AI processor calculates a remaining amount of workout, which represents a difference between a target amount of workout of a target muscle and an accumulated amount of workout of the target muscle when the user does not perform the entire workout sequence. In this case, referring to FIG. 7, the display 710 may display a workout progress status 740. The workout progress status 740 includes at least one of a target amount of workout 740a, an accumulated amount of workout 740b, and a remaining amount of workout 740c. The display 710 displays in real time one or more muscles 730a, 730b, 730c, and 730d activated when the user works out and the accumulated amount of workout 740b in numerical values. According to another embodiment of the present disclosure, the display 710 may display the one or more activated muscles 730a, 730b, 730c, and 730d and the accumulated amount of workout 740b of each of the one or more activated muscles 730a, 730b, 730c, and 730 on a character. In addition, the display 710 may further display the workout progress status 740 on the character to display the one or more activated muscles 730a, 730b, 730c, and 730d activated through workout and the accumulated amount of workout 740b of each of the one or more activated muscles 730a, 730b, 730c, and 730 in numerical values on the character.

Methods according to embodiments may be recorded on a computer-readable recording medium by being implemented in a form of program commands executed by using various computers. The computer-readable recording medium may include at least one of a program command, a data file, or a data structure. The program commands recorded in the computer-readable recording medium may be specially designed or well known to one of ordinary skill in the computer software field.

While one or more embodiments have been described with reference to the figures, it will be understood by those of ordinary skill in the art that various changes in form and details may be made therein without departing from the spirit and scope of the disclosure as defined by the following claims.

## Claims

1. An artificial intelligence (Al) workout guide device comprising:
a storage storing one or more muscles activated when fitness equipment is used, a contribution of each of the one or more muscles, and an workout trajectory guide generated when the fitness equipment is used accurately with a correct posture;
a sensor configured to detect an workout trajectory corresponding to movement of a user moving the fitness equipment;
an AI processor configured to determine whether a deviation between the workout trajectory detected by the sensor when the fitness equipment is moved in at least one direction and the workout trajectory guide exceeds a preset value; and
a workout guider configured to, when the deviation exceeds the preset value, guide to adjust an workout load of the fitness equipment or an workout posture.

2. The AI workout guide device of claim 1, further comprising: a display configured to, based on the contribution, display a load applied to each of the one or more muscles activated when a user uses the fitness equipment.

3. The AI workout guide device of claim 1, further comprising: a display configured to display in real time information about a state of contraction or relaxation of each of one or more muscles activated when the fitness equipment is used.

4. The AI workout guide device of claim 3, further comprising: a display configured to display in real time a contraction guide or relaxation guide with respect to a major muscle activated when a user uses the fitness equipment, based on the workout trajectory detected by the sensor and the workout trajectory guide.

5. The AI workout guide device of claim 1, further comprising: a display configured to display a necessary breathing guide as an indicator according to contraction or relaxation of a major muscle among one or more muscles activated when the fitness equipment is used.

6. The AI workout guide device of claim 1, wherein the sensor is configured to further detect an angle of a joint when the user moves the fitness equipment.

7. The AI workout guide device of claim 6, further comprising: a display configured to, based on the detected angle of the joint, display in real time information about a state of contraction or relaxation of each of one or more muscles activated when the fitness equipment is used.

8. The AI workout guide device of claim 1, wherein the workout trajectory guide is generated by a machine learning processor analyzing user big data.

9. The AI workout guide device of claim 6, wherein the workout guider is configured to guide a workout posture to be adjusted when a shape of an workout trajectory corresponding to the angle of the joint in a certain range detected by the sensor is different from a shape of the workout trajectory of the previously stored workout trajectory guide.

10. An artificial intelligence (Al) workout guide method comprising:
detecting, by a sensor, an workout trajectory corresponding to movement of a user moving fitness equipment;
determining, by an AI processor, whether the workout trajectory detected by the sensor when the fitness equipment is moved in at least one direction matches an workout trajectory guide previously stored in a storage and generated when the fitness equipment is used appropriately according to usage standards; and
guiding, by a workout guider, an workout load of the fitness equipment or an workout posture to be adjusted when a deviation between the workout trajectory detected and the workout trajectory guide exceeds a preset value.

11. The AI workout guide method of claim 10, further comprising: based on the contribution, displaying, by the display, a load applied to each of one or more muscles activated when a user uses the fitness equipment.

12. The AI workout guide method of claim 10, further comprising: displaying in real time, by the display, information about a state of contraction or relaxation of each of one or more muscles activated when the fitness equipment is used.

13. The AI workout guide method of claim 10, further comprising: displaying as indicators, by the display, an exhalation in a direction in which a major muscle contracts and an inhalation in a direction in which the major muscle relaxes, among one or more muscles activated when the fitness equipment is used.

14. The AI workout guide method of claim 10, further comprising: further detecting, by the sensor, an angle of a joint when the user moves the fitness equipment, and based on the detected angle of the joint, displaying in real time, by the display, information about a state of contraction or relaxation of each of one or more muscles activated when the fitness equipment is used.

15. A computer-readable recording medium storing commands for enabling a computing device to perform an artificial intelligence (Al) workout guide method comprising:
detecting, by a sensor, an workout trajectory corresponding to movement of a user moving fitness equipment;
determining, by an AI processor, whether the workout trajectory detected by the sensor when the fitness equipment is moved in at least one direction matches an workout trajectory guide previously stored in a storage and generated when the fitness equipment is used appropriately according to usage standards; and
guiding, by a workout guider, an workout load of the fitness equipment or an workout posture to be adjusted when a deviation between the workout trajectory detected and the workout trajectory guide exceeds a preset value.
